# EUROPEAN PATENT APPLICATION

(11) **EP 4 593 020 A1**
(43) Date of publication of application: **30.07.2025**
(21) Application number: 23867961.7
(22) Date of filing: 24.08.2023
(51) Int. Cl.: G16C 60/00

(54) **DESIGN ASSISTANCE DEVICE, DESIGN ASSISTANCE METHOD, PROGRAM, AND INFORMATION PROCESSING SYSTEM**

(30) Priority: 20.09.2022 JP 2022149308
(71) Applicant: Resonac Corporation, Tokyo 105-7325 (JP)
(72) Inventor: AONUMA, Naoto, Tokyo 105-8518 (JP); NISHINO, Shogo, Tokyo 105-8518 (JP); TAKEMOTO, Shimpei, Tokyo 105-8518 (JP); OKUNO, Yoshishige, Tokyo 105-8518 (JP)
(74) Representative: Strehl & Partner mbB
(86) International application number: PCT/JP2023/030551
(87) International publication number: WO 2024/062837

(57) **Abstract**

A design assistance device, which is configured to assist design of a material in which a plurality of raw materials are to be formulated, by using a machine learning model that has learned a correspondence relationship between design condition information of a material, in which a plurality of raw materials are to be formulated, and property information of the material, the design assistance device including a raw material information storage configured to store information of names and attributes of a plurality of registered raw materials; a registration reception unit configured to receive an input of information of a name and an attribute of a raw material to be newly registered, and store the received information in the raw material information storage; and a feature generation unit configured to generate a feature of the raw material that is inputtable to the machine learning model, the feature being generated from information that is selected as the design condition information and is information of attributes of a plurality of raw materials stored in the raw material information storage.

## Description

### TECHNICAL FIELD

The present disclosure relates to a design assistance device, a design assistance method, a program, and an information processing system.

### BACKGROUND ART

Material design assistance devices have been known, which use a machine learning model that learns an experimental dataset (training dataset) recording a correspondence relationship between material design condition information (e.g., formulation amounts of raw materials, process conditions, and the like) and material property information, thereby predicting properties of a material including a plurality of raw materials included in the training dataset.

For example, Patent Literature 1 describes a material property prediction system for predicting material properties by processing case data including a plurality of records each formed by a material composition, experimental conditions, and material properties.

### CITATION LIST

### PATENT LITERATURE

Patent Literature 1: Japanese Laid-Open Patent Publication No. 2021-47627

### SUMMARY OF THE INVENTION

### PROBLEM TO BE SOLVED BY THE INVENTION

It would be convenient if existing material design assistance devices could predict properties of a material in which a raw material not included in a training dataset is to be formulated, thereby assisting material design. Patent Literature 1 does not describe such a matter.

It is an object of the present disclosure to provide a design assistance device, a design assistance method, a program, and an information processing system that assist material design by predicting properties of a material in which a raw material not included in a training dataset is to be formulated.

### MEANS FOR SOLVING THE PROBLEM

The present disclosure includes configurations as presented below.

[1] A design assistance device, which is configured to assist design of a material in which a plurality of raw materials are to be formulated, by using a machine learning model that has learned a correspondence relationship between design condition information of a material, in which a plurality of raw materials are to be formulated, and property information of the material, the design assistance device including:
   a raw material information storage configured to store information of names and attributes of a plurality of registered raw materials;
   a registration reception unit configured to receive an input of information of a name and an attribute of a raw material to be newly registered, and store the received information in the raw material information storage; and
   a feature generation unit configured to generate a feature of the raw material that is inputtable to the machine learning model, the feature being generated from information that is selected as the design condition information and is information of attributes of a plurality of raw materials stored in the raw material information storage.
[2] The design assistance device according to [1], in which
   the registration reception unit is configured to prohibit new registration of a raw material that matches information of a molecular structure of the raw material stored in the raw material information storage.
[3] The design assistance device according to [1], in which
   the information of the attribute of the raw material includes information of use of the raw material, and
   the registration reception unit is configured to prohibit new registration of a raw material that matches information of a molecular structure of the raw material and information of the use of the raw material, which are stored in the raw material information storage.
[4] The design assistance device according to [3], in which
   the registration reception unit is configured to prohibit new registration of a raw material that matches the name of the raw material stored in the raw material information storage.
[5] The design assistance device according to any one of [1] to [4], in which
   the registration reception unit is configured to receive an input of information of a molecular structure of a raw material in accordance with a SMILES notation, a SMARTS notation, or an InChI notation, as the information of the attribute of the raw material to be newly registered.
[6] The design assistance device according to any one of [1] to [4], in which
   the registration reception unit is configured to receive an input of information of a molecular structure of a raw material in an MOL format, an SDF format, a PDB format, or a CIF format, as the information of the attribute of the raw material to be newly registered.
[7] The design assistance device according to any one of [1] to [6], in which
   the feature generation unit is configured to generate the feature of the raw material from information of a molecular structure of the raw material by using a fingerprint method.
[8] The design assistance device according to any one of [1] to [7], in which
   the feature generation unit is configured to generate the feature of the raw material by calculating a physical property value of a molecule from a molecular structure of the raw material.
[9] The design assistance device according to any one of [1] to [8], in which
   the information of the attribute whose input is received by the registration reception unit includes a physical property value of the raw material.
[10] The design assistance device according to any one of [1] to [9], in which
   the design assistance device includes
   a design condition input reception unit configured to receive an input of the design condition information that includes information of a plurality of raw materials stored in the raw material information storage, and
   a material property prediction unit configured to predict a property of the material using the machine learning model in accordance with the design condition information whose input has been received.
[11] The design assistance device according to any one of [1] to [9], further including:
   a required property input reception unit configured to receive an input of required property information of the material;
   a range information input reception unit configured to receive an input of range information of the design condition information that includes information of a plurality of raw materials stored in the raw material information storage; and
   a design condition proposal unit configured to propose a candidate of the design condition information that satisfies the required property information, from a result obtained by predicting a property of the material using the machine learning model in accordance with the design condition information within a range of the range information.
[12] The design assistance device according to [10] or [11], further including:
   a display controller configured to display a predicted property of the material, or a proposed candidate of the design condition information.
[13] A design assistance method, in which a computer assists design of a material in which a plurality of raw materials are to be formulated, by using a machine learning model that has learned a correspondence relationship between design condition information of a material, in which a plurality of raw materials are to be formulated, and property information of the material, the design assistance method including:
   a registration reception step of receiving an input of information of a name and an attribute of a raw material to be newly registered, and registering the received information in a raw material information storage that stores information of names and attributes of a plurality of registered raw materials; and
   a feature generation step of generating a feature of the raw material that is inputtable to the machine learning model, the feature being generated from information that is selected as the design condition information and is information of attributes of a plurality of raw materials stored in the raw material information storage.
[14] A program causing a computer to execute, the computer assisting design of a material in which a plurality of raw materials are to be formulated, by using a machine learning model that has learned a correspondence relationship between design condition information of a material, in which a plurality of raw materials are to be formulated, and property information of the material:
   a registration reception procedure of receiving an input of information of a name and an attribute of a raw material to be newly registered, and registering the received information in a raw material information storage that stores information of names and attributes of a plurality of registered raw materials; and
   a feature generation procedure of generating a feature of the raw material that is inputtable to the machine learning model, the feature being generated from information that is selected as the design condition information and is information of attributes of a plurality of raw materials stored in the raw material information storage.
[15] An information processing system, in which a plurality of computers assist design of a material in which a plurality of raw materials are to be formulated, by using a machine learning model that has learned a correspondence relationship between design condition information of a material, in which a plurality of raw materials are to be formulated, and property information of the material, the design assistance system including:
   a raw material information storage configured to store information of names and attributes of a plurality of registered raw materials;
   a registration reception unit configured to receive an input of information of a name and an attribute of a raw material to be newly registered, and store the received information in the raw material information storage; and
   a feature generation unit configured to generate a feature of the raw material that is inputtable to the machine learning model, the feature being generated from information that is selected as the design condition information and is information of attributes of a plurality of raw materials stored in the raw material information storage.

### ADVANTAGEOUS EFFECTS OF THE INVENTION

According to the present disclosure, it is possible to provide a design assistance device, a design assistance method, a program, and an information processing system that assist material design by predicting properties of a material in which a raw material not included in a training dataset is to be formulated.

### BRIEF DESCRIPTION OF THE DRAWINGS

[FIG. 1] FIG. 1 is a configuration diagram of an example of an information processing system according to the present embodiment.
[FIG. 2] FIG. 2 is a hardware configuration diagram of an example of a computer according to the present embodiment.
[FIG. 3] FIG. 3 is a functional configuration diagram of an example of the information processing system according to the present embodiment.
[FIG. 4] FIG. 4 is an image diagram of an example of a new raw material registration screen according to the present embodiment.
[FIG. 5] FIG. 5 is an image diagram of an example of the new raw material registration screen according to the present embodiment.
[FIG. 6] FIG. 6 is an image diagram of an example of a registered raw material list screen.
[FIG. 7] FIG. 7 is a flowchart illustrating an example of a process of prohibiting registration of a new raw material having an equivalent molecular structure.
[FIG. 8] FIG. 8 is a flowchart illustrating an example of the process of prohibiting registration of a new raw material having an equivalent molecular structure.
[FIG. 9] FIG. 9 is a flowchart illustrating an example of the process of prohibiting registration of a new raw material having an equivalent molecular structure.
[FIG. 10] FIG. 10 is a flowchart illustrating an example of a process of assistance of material design through direct problem analysis.
[FIG. 11A] FIG. 11A is a configuration diagram of an example of design condition information of a material.
[FIG. 11B] FIG. 11B is a configuration diagram of an example of predicted property information of a material.
[FIG. 12] FIG. 12 is an explanatory diagram of an example of a process of generating a feature of a raw material to be formulated in a material.
[FIG. 13] FIG. 13 is a flowchart illustrating an example of a process of assistance of material design through inverse problem analysis.
[FIG. 14] FIG. 14 is a configuration diagram of an example of required property information of a material.
[FIG. 15] FIG. 15 is a configuration diagram of an example of range information of design condition information of a material.
[FIG. 16A] FIG. 16A is a configuration diagram of an example of proposed material information.
[FIG. 16B] FIG. 16B is a configuration diagram of an example of the proposed material information.

### DESCRIPTION OF THE EMBODIMENTS

Next, embodiments of the present invention will be described in detail. The present invention is not limited to the following embodiments.

### [First Embodiment]

### <System Configuration>

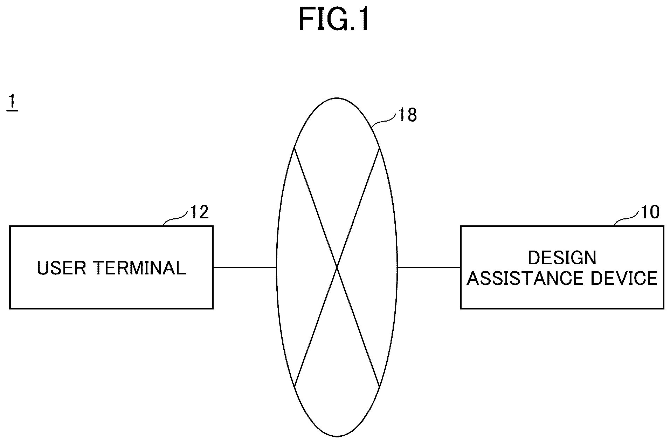

FIG. 1 is a configuration diagram of an example of an information processing system according to the present embodiment. An information processing system 1 of FIG. 1 includes a design assistance device 10 and a user terminal 12. The design assistance device 10 and the user terminal 12 are connected so as to enable data communication via a communication network 18, such as a local area network (LAN), the Internet, or the like.

The user terminal 12 is an information processing terminal that is operated by an operator, such as a PC, a tablet terminal, a smartphone, or the like. The user terminal 12 is configured to display, on a display device, a screen configured to receive an input of information from an operator, and receive an input of information from the operator. Also, the user terminal 12 is configured to transmit, to the design assistance device 10, the information whose input has been received from the operator, and cause the design assistance device 10 to execute a process of assistance of design of a material in which a plurality of raw materials are to be formulated. The user terminal 12 is configured to receive the information of an execution result of the process of the design assistance device 10, and display the received information on the display device for confirmation by the operator.

The design assistance device 10 is an information processing device, such as a PC or the like, that is configured to assist, as described below, design of a material in which a plurality of raw materials are to be formulated. The design assistance device 10 is configured to perform a process of an inverse problem analysis for proposing a candidate of the design condition information of a material that satisfies required property information, and a process of a direct problem analysis for predicting a property of a material in accordance with the design condition information. The design assistance device 10 is configured to assist design of a material in which a plurality of raw materials are to be formulated, by performing the process of inverse problem analysis and the process of direct problem analysis.

The design assistance device 10 can utilize a mathematical model, such as a machine learning model (trained machine learning model) that has learned a correspondence relationship between: design condition information of a material in which a plurality of raw materials are to be formulated; and property information of the material. A method of the machine learning may be a supervised learning method, such as linear, generalized linear (lasso, ridge, elastic net, or logistic), partial least squares, Kernel ridge, a Gaussian process, a k-nearest neighbor algorithm, a decision tree, a random forest, AdaBoost, bagging, gradient boosting, a support vector machine, a neural network, or the like.

The process of the inverse problem analysis includes inputting the required property information and range information of the design condition information, and generating exhaustive search points in the design condition information within a range at random or at a predetermined step width. Also, the process of the inverse problem analysis can predict a property of a material corresponding to each of the exhaustive search points by using the trained machine learning model, and extract the exhaustive search points of a property that satisfies the required property information, thereby obtaining a candidate of the design condition information that satisfies desired required property information.

When the trained machine learning model predicts a property of a material in which a plurality of raw materials are to be formulated, a compositional ratio, a formulation amount, or the like of each raw material to be formulated in the material is input. The design assistance device 10 creates a feature (explanatory variable) of a raw material to be input to the trained machine learning model, in accordance with information obtained by expressing the molecular structure of the raw material with numerical values, and the compositional ratio and the formulation amount of the raw material. The following description will be made taking, as an example, the formulation amount of the raw material.

An example of the method of expressing the molecular structure of a raw material with numerical values is Extended Circular FingerPrints (hereinafter referred to as ECFP). The ECFP is a method of expressing a structural feature of a molecule by extracting types and counts of all partial structures from the molecular structure of a raw material, and expressing the types and the counts as a vector (column: type, value: count).

An input of the information of the molecular structure of the raw material can be performed using a notation method, i.e., the simplified molecular input line entry system (hereinafter referred to as SMILES) notation, the SMARTS notation, or the InChI notation. The ECFP can express the molecular structure of a raw material with numerical values, for example, by inputting information of the raw material expressed in the SMILES notation into an existing library.

Another example of the method of expressing the molecular structure of a raw material with numerical values is a method of expressing a structural feature of a molecule by extracting a physical property value (e.g., a molecular weight, the number of radical charges, the number of valence electrons, or the like), which can be calculated from the molecular structure of the raw material, and expressing the physical property value as a vector (column: type, value: numerical value). The physical property value can be extracted, for example, by inputting information of a raw material expressed in the SMILES notation into an existing library.

The design assistance device 10 receives information input by an operator into the user terminal 12, and executes a process of assistance of design of a material. The design assistance device 10 transmits result information of the process to the user terminal 12, and causes the user terminal 12 to display the result information of the process.

The information processing system 1 of FIG. 1 can be implemented by the design assistance device 10 having a Web server function, and the user terminal 12 configured to execute a Web application by a Web browser function. The information processing system 1 of FIG. 1 may be implemented by an application installed in the user terminal 12 performing the process in cooperation with a program installed in the design assistance device 10.

The information processing system 1 of FIG. 1 is just an example, and there are various system configuration examples in accordance with applications and purposes. For example, the design assistance device 10 may be implemented by a plurality of computers or may be implemented as a cloud computing service. Also, the information processing system 1 of FIG. 1 may be implemented as a stand-alone computer.

### <Hardware Configuration>

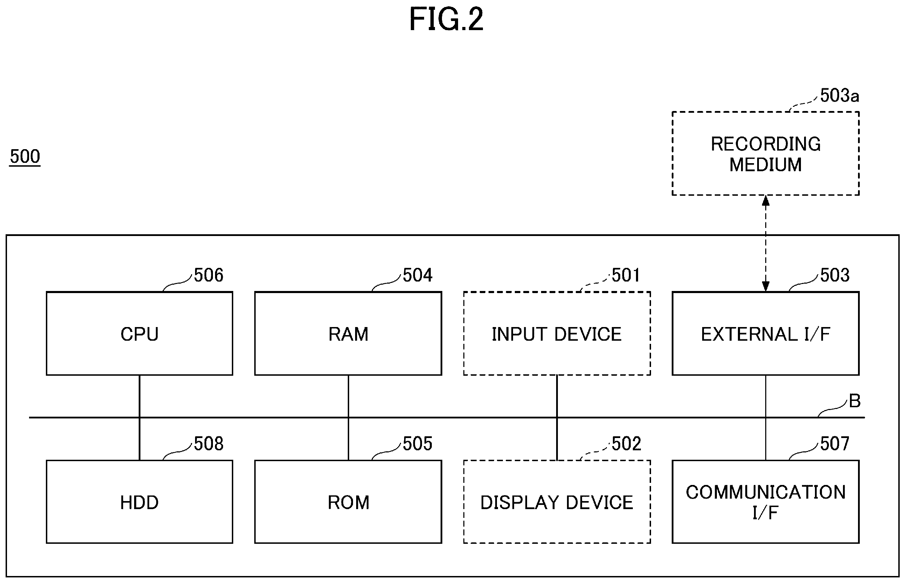

The design assistance device 10 and the user terminal 12 of FIG. 1 are implemented, for example, by a computer 500 having a hardware configuration as illustrated in FIG. 2.

FIG. 2 is a hardware configuration diagram of an example of a computer according to the present embodiment. The computer 500 of FIG. 2 includes an input device 501, a display device 502, an external I/F 503, a RAM 504, a ROM 505, a CPU 506, a communication I/F 507, an HDD 508, and the like, and these are connected to each other through a bus B. The input device 501 and the display device 502 may be connected for use.

The input device 501 is a touch panel, an operation key, a button, a keyboard, a mouse, or the like, which is used by a user to input various signals. The display device 502 is formed, for example, by a display, such as a liquid crystal or organic EL display configured to display a screen, and a speaker configured to output sound data, such as voices, sounds, and the like. The communication I/F 507 is an interface for data communication performed by the computer 500.

The HDD 508 is an example of a non-volatile storage device that stores programs and data. The programs and data stored in the HDD 508 are: OS that is basic software controlling the entire computer 500; applications that provide various functions on the OS; and the like. The computer 500 may utilize, instead of the HDD 508, a drive device using a flash memory as a storage medium (e.g., a solid state drive (SSD) or the like).

The external I/F 503 is an interface with an external device. The external device is a recording medium 503a or the like. Thus, the computer 500 can read from and/or write in the recording medium 503a via the external I/F 503. The recording medium 503a is a flexible disk, a CD, a DVD, an SD memory card, a USB memory, or the like.

The ROM 505 is an example of a non-volatile semiconductor memory (storage device) that is configured to retain programs and data even if power is turned off. The ROM 505 stores programs and data performed upon start-up of the computer 500, such as BIOS, OS setting, network setting, and the like. The RAM 504 is an example of a volatile semiconductor memory (storage device) that is configured to temporarily retain programs and data.

The CPU 506 is a computing device configured to implement controls and functions of the entire computer 500 by reading out programs and data on the RAM 504 from a storage device, such as the ROM 505, the HDD 508, or the like, and performing processes. By executing programs, the computer 500 according to the present embodiment can implement the below-described various functions of the design assistance device 10 and the user terminal 12.

### <Functional Configuration>

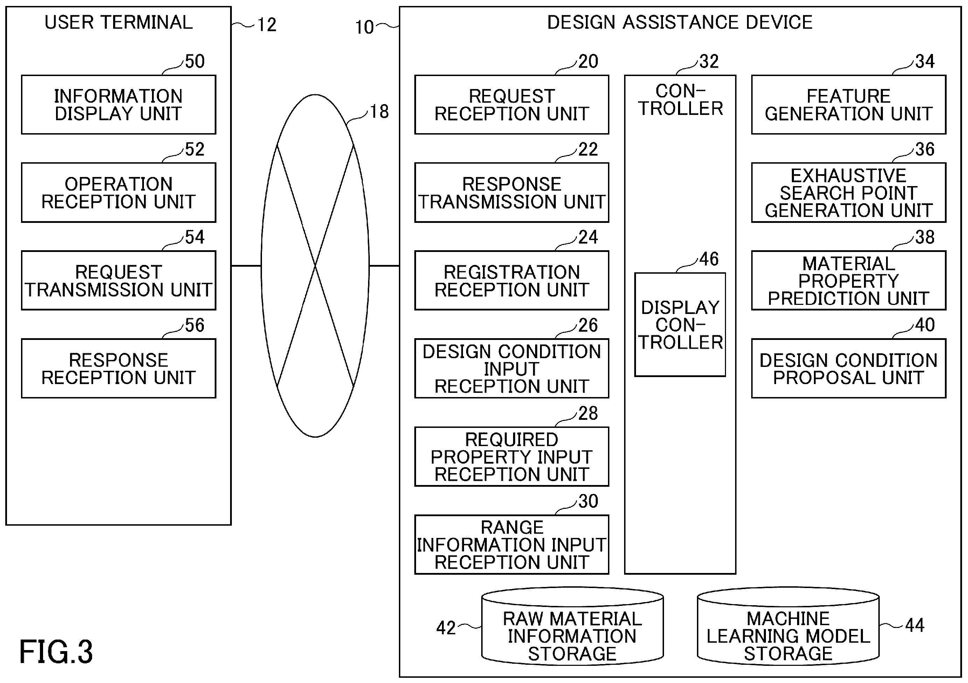

A functional configuration of the information processing system 1 according to the present embodiment will be described below. FIG. 3 is a functional configuration diagram of an example of the information processing system according to the present embodiment. The configuration diagram of FIG. 3 appropriately omits components that are unnecessary for the description of the present embodiment.

The design assistance device 10 of the information processing system 1 illustrated in FIG. 3 includes a request reception unit 20, a response transmission unit 22, a registration reception unit 24, a design condition input reception unit 26, a required property input reception unit 28, a range information input reception unit 30, a controller 32, a feature generation unit 34, an exhaustive search point generation unit 36, a material property prediction unit 38, a design condition proposal unit 40, a raw material information storage 42, a machine learning model storage 44, and a display controller 46. The user terminal 12 includes an information display unit 50, an operation reception unit 52, a request transmission unit 54, and a response reception unit 56.

The information display unit 50 is configured to display, on the display device 502, a screen configured to receive an input of information from an operator and information of an execution result of the process of the design assistance device 10. The operation reception unit 52 is configured to receive an operation of an operator, such as an input of information or the like. The request transmission unit 54 is configured to transmit, to the design assistance device 10, a request for a process in accordance with the input of information from the operator. The response reception unit 56 is configured to receive, from the design assistance device 10, a response to the request for the process transmitted by the request transmission unit 54.

The request reception unit 20 is configured to receive a request for a process from the user terminal 12. The response transmission unit 22 is configured to respond to the execution result of the process in accordance with the request for the process. The registration reception unit 24 is configured to receive an input of information of a name and an attribute of a raw material to be newly registered, and store the received information in the raw material information storage 42. The raw material information storage 42 stores information of names and attributes of a plurality of registered raw materials.

The design condition input reception unit 26 is configured to receive, from an operator, an input of design condition information including information of the plurality of raw materials stored in the raw material information storage 42. The required property input reception unit 28 is configured to receive, from an operator, an input of required property information of a material in which a plurality of raw materials are to be formulated. The range information input reception unit 30 is configured to receive an input of range information of design condition information including information of the plurality of raw materials stored in the raw material information storage 42.

As described below, the feature generation unit 34 is configured to generate a feature of a raw material that is inputtable to the trained machine learning model, from information of the attributes of the raw material. The exhaustive search point generation unit 36 is configured to generate a predetermined number (e.g., 1,000) of exhaustive search points at random or at a predetermined step width within a range of range information of the design condition information. The exhaustive search point is a combination of compositional ratios or formulation amounts of a plurality of raw materials to be formulated in a material. The formulation amount of each of the raw materials is selected, for example, from within a range indicated by a lower limit and an upper limit of the formulation amount.

The material property prediction unit 38 is configured to predict a property of a material by using a trained machine learning model in accordance with the design condition information whose input has been received. The design condition proposal unit 40 is configured to propose a candidate of design condition information, which satisfies the input required property information, by using a trained machine learning model. The design condition proposal unit 40 predicts a property of a material corresponding to each of the exhaustive search points by using the trained machine learning model, and extracts an exhaustive search point of a property that satisfies the required property information, thereby obtaining a candidate of design condition information that satisfies desired required property information.

The machine learning model storage 44 is configured to store, in a trained machine learning model, a correspondence relationship between design condition information of a material and property information of the material. The design condition information of the material includes information, such as types and formulation amounts of raw materials, process conditions, and the like. The process conditions are, for example, the process temperature and process time of a process, such as a thermal process or the like. The property information of the material is, for example, a viscosity, a glass transition temperature, a molecular weight, an acid value, and the like.

The controller 32 is configured to control the request reception unit 20, the response transmission unit 22, the registration reception unit 24, the design condition input reception unit 26, the required property input reception unit 28, the range information input reception unit 30, the feature generation unit 34, the exhaustive search point generation unit 36, the material property prediction unit 38, the design condition proposal unit 40, and the display controller 46. The display controller 46 is configured to perform control in a manner that a material property predicted by the material property prediction unit 38 or a candidate of the design condition information proposed by the design condition proposal unit 40 is displayed on the user terminal 12.

The configuration diagram of FIG. 3 is just an example. The configuration of the information processing system 1 according to the present embodiment can be implemented in various configurations. For example, the raw material information storage 42 and the machine learning model storage 44 may be included in a storage device, a computer, a cloud storage, or the like, which is configured to perform data communication with the design assistance device 10.

### <Process>

### <<New Raw Material Registration>>

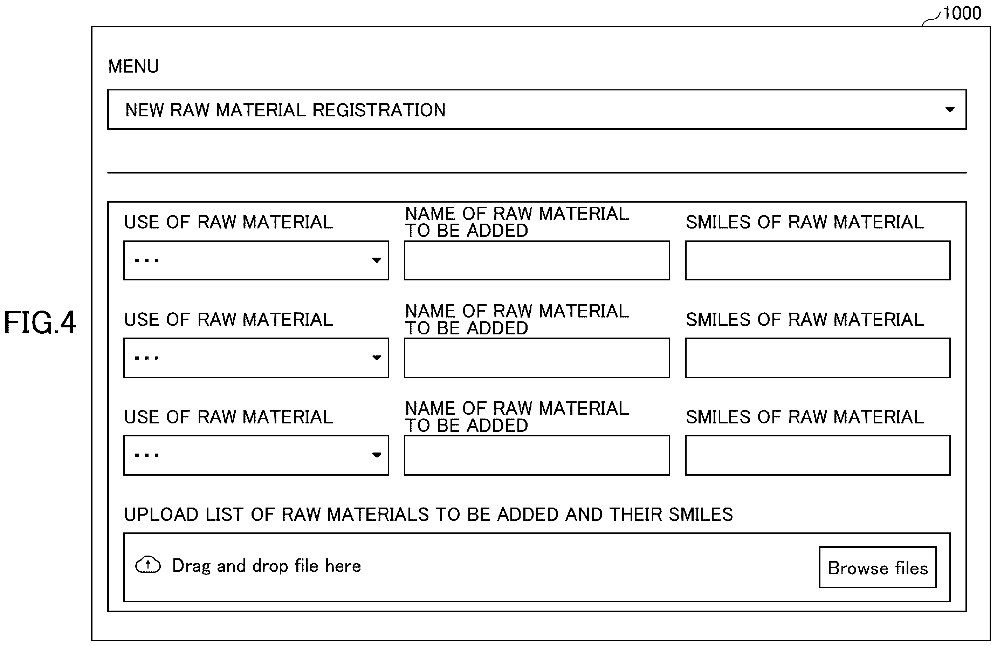

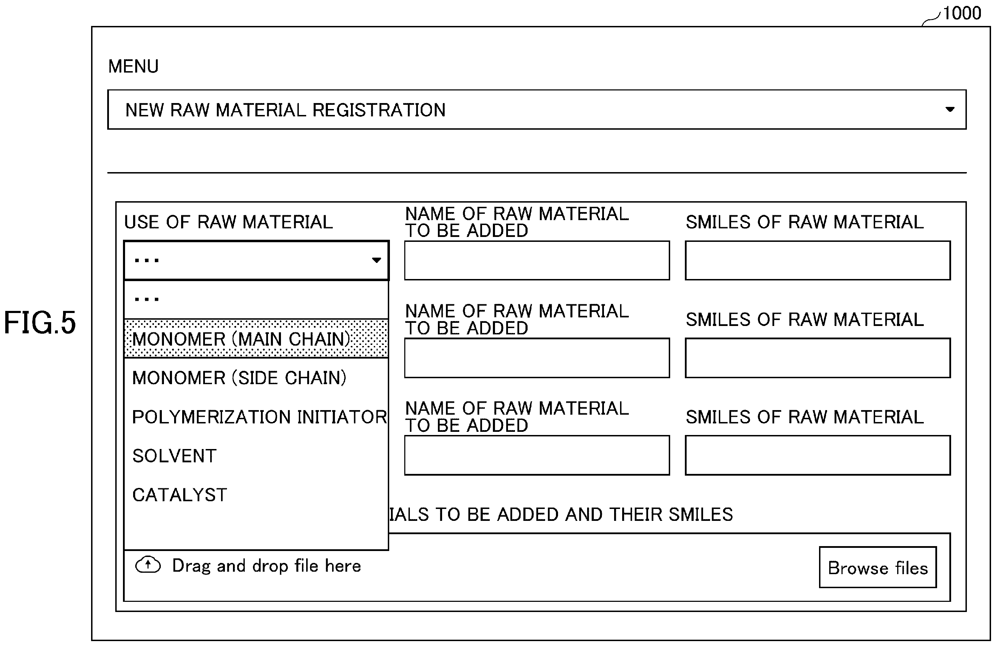

The information processing system 1 according to the present embodiment causes the user terminal 12 to display, for example, a new raw material registration screen 1000 as illustrated in FIGS. 4 and 5 in response to an operator selecting a menu for registration of a new raw material.

FIGS. 4 and 5 are image diagrams of an example of a new raw material registration screen according to the present embodiment. The new raw material registration screen 1000 of FIG. 4 is an example of a screen for an input of the use of a raw material to be newly registered, the name of a raw material to be added, and the SMILES of the raw material. The name of the raw material to be added is the name of the raw material to be newly registered, and is an example of information by which an operator identifies a raw material. The use of the raw material and the SMILES of the raw material are examples of information of the attributes of the raw material.

The use of the raw material is an example of information indicating the use of the raw material, such as a monomer (main chain), a monomer (side chain), a polymerization initiator, a solvent, a catalyst, or the like, for example, as illustrated in FIG. 5. The new raw material registration screen 1000 of FIG. 5 illustrates an example in which an operator selects a single use of a raw material from a plurality of uses of the raw material.

The SMILES of the raw material is an example of information of the molecular structure of a raw material to be newly registered. Although the new raw material registration screen 1000 of FIGS. 4 and 5 illustrates an example in which information of the molecular structure of the raw material to be newly registered is input in accordance with a SMILES notation, the information may be input in accordance with a SMARTS notation or an InChI notation. Also, information of the molecular structure of a raw material to be newly registered may be input to the new raw material registration screen 1000 of FIGS. 4 and 5 in an MOL format, an SDF format, a PDB format, or a CIF format, which is a file format for expressing a molecular structure. The new raw material registration screen 1000 of FIGS. 4 and 5 can receive an input of information of a raw material to be added, by uploading a list of the use of the raw material to be added, the name of the raw material to be added, and the SMILES of the raw material.

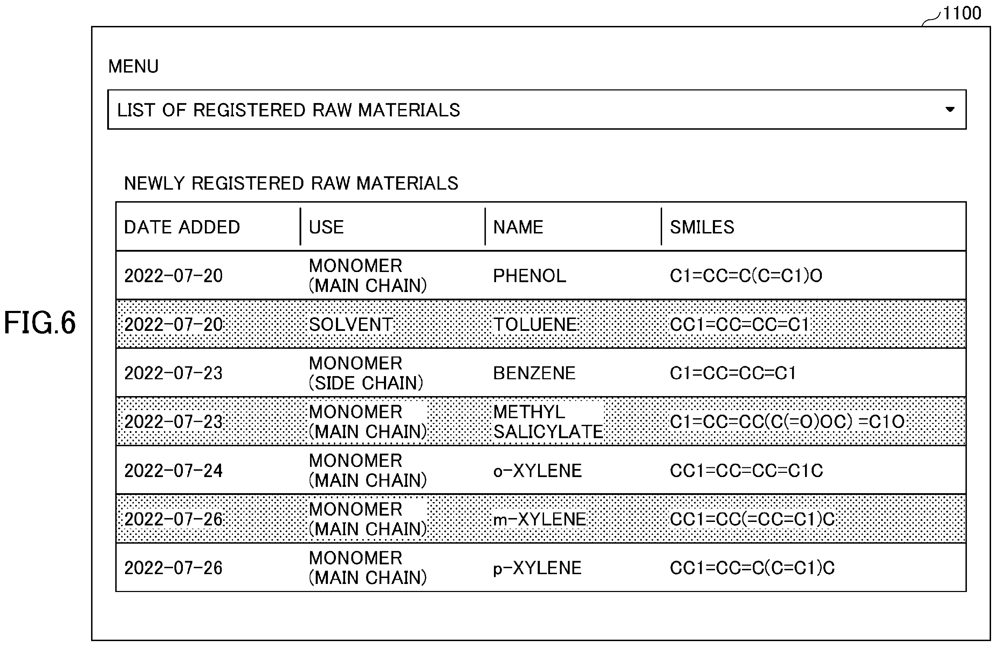

By selecting a menu for displaying the registered raw material, for example, the operator can display, on the user terminal 12, a registered raw material list screen 1100 as illustrated in FIG. 6. FIG. 6 is an image diagram of an example of a registered raw material list screen. The registered raw material list screen 1100 of FIG. 6 is an example of a screen that displays a list of the date added, use, name, and SMILES of the raw material that has been newly registered from the new raw material registration screen 1000, as illustrated in FIGS. 4 and 5, and has been stored in the raw material information storage 42.

According to the information processing system 1 according to the present embodiment, duplicate registration of a molecular structure equivalent to that of the raw material stored in the raw material information storage 42 is prohibited in the following manner.

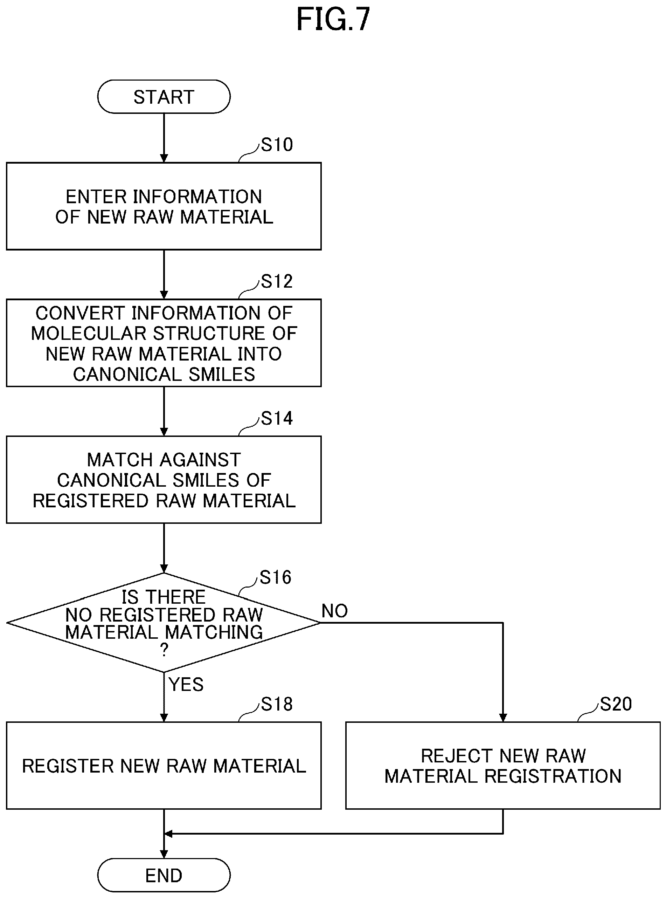

FIG. 7 is a flowchart illustrating an example of a process of prohibiting registration of a new raw material having an equivalent molecular structure. In step S10, the registration reception unit 24 of the design assistance device 10 receives an input of information of a new raw material from the new raw material registration screen 1000 illustrated in FIGS. 4 and 5.

In step S12, the registration reception unit 24 converts information of a molecular structure included in the information of the new raw material, whose input has been received, into canonical SMILES of the new raw material. In step S14, the registration reception unit 24 performs matching of the canonical SMILES of the new raw material against the canonical SMILES of the raw material registered in the raw material information storage 42. The matching in terms of the canonical SMILES is just an example, and information used for matching may be any information as long as uniqueness of a raw material can be used for matching.

In step S16, the registration reception unit 24 determines whether or not there is a raw material registered in the raw material information storage 42 whose canonical SMILES matches that of the new raw material. If there is no raw material registered in the raw material information storage 42 whose canonical SMILES matches that of the new raw material, the registration reception unit 24 determines that the new raw material does not have a molecular structure equivalent to that of the raw material stored in the raw material information storage 42, and registers the new raw material in the raw material information storage 42 in step S18.

On the other hand, if there is a raw material registered in the raw material information storage 42 whose canonical SMILES matches that of the new raw material, the registration reception unit 24 determines that the new raw material has a molecular structure equivalent to that of the raw material stored in the raw material information storage 42, and performs the process of step S20. In step S20, the registration reception unit 24 rejects registration of the new raw material in the raw material information storage 42 in order to prohibit duplicate registration of the new raw material having a molecular structure equivalent to that of the raw material stored in the raw material information storage 42.

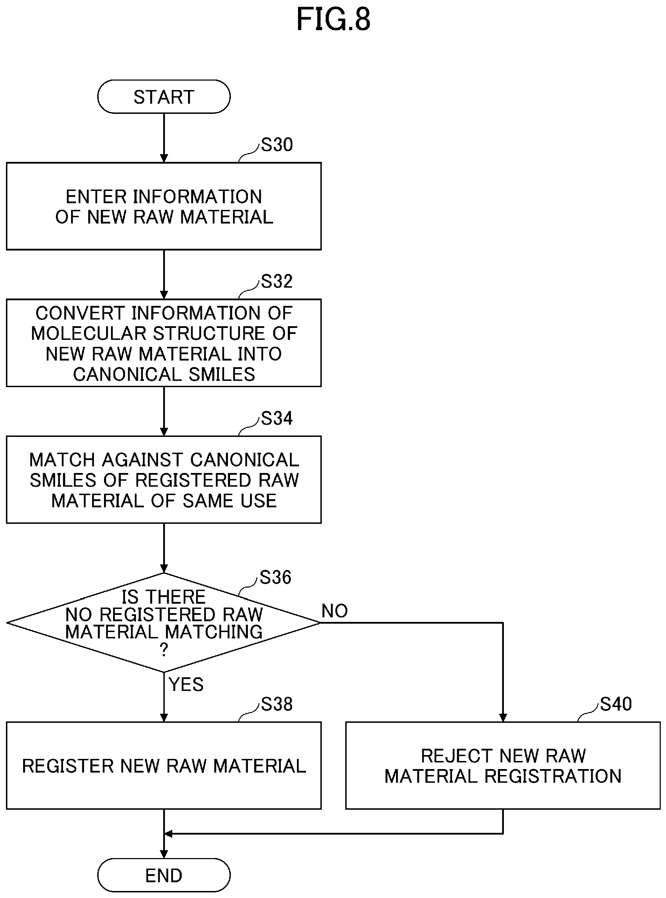

FIG. 8 is a flowchart illustrating an example of a process of prohibiting registration of a new raw material having an equivalent molecular structure. In step S30, the registration reception unit 24 of the design assistance device 10 receives an input of information of a new raw material from the new raw material registration screen 1000 illustrated in FIGS. 4 and 5.

In step S32, the registration reception unit 24 converts, into canonical SMILES of the new raw material, information of a molecular structure included in the information of the new raw material whose input has been received. In step S34, the registration reception unit 24 performs matching of the canonical SMILES of the new raw material against the canonical SMILES of the raw material registered in the raw material information storage 42. The matching in terms of the canonical SMILES is just an example, and information used for matching may be any information as long as uniqueness of a raw material can be used for matching.

In step S36, the registration reception unit 24 determines whether or not there is a raw material registered in the raw material information storage 42 whose use is the same as that of the new raw material and whose canonical SMILES matches that of the new raw material.

If there is no raw material registered in the raw material information storage 42 whose use is the same as that of the new raw material and whose canonical SMILES matches that of the new raw material, the registration reception unit 24 registers the new raw material in the raw material information storage 42 in step S38.

On the other hand, if there is a raw material registered in the raw material information storage 42 whose use is the same as that of the new raw material and whose canonical SMILES matches that of the new raw material, the registration reception unit 24 determines that the new raw material has a molecular structure equivalent to that of the raw material of the same use stored in the raw material information storage 42, and performs the process of step S40. In step S40, the registration reception unit 24 rejects registration of the new raw material in the raw material information storage 42 in order to prohibit duplicate registration of the new raw material having a molecular structure equivalent to that of the raw material of the same use stored in the raw material information storage 42.

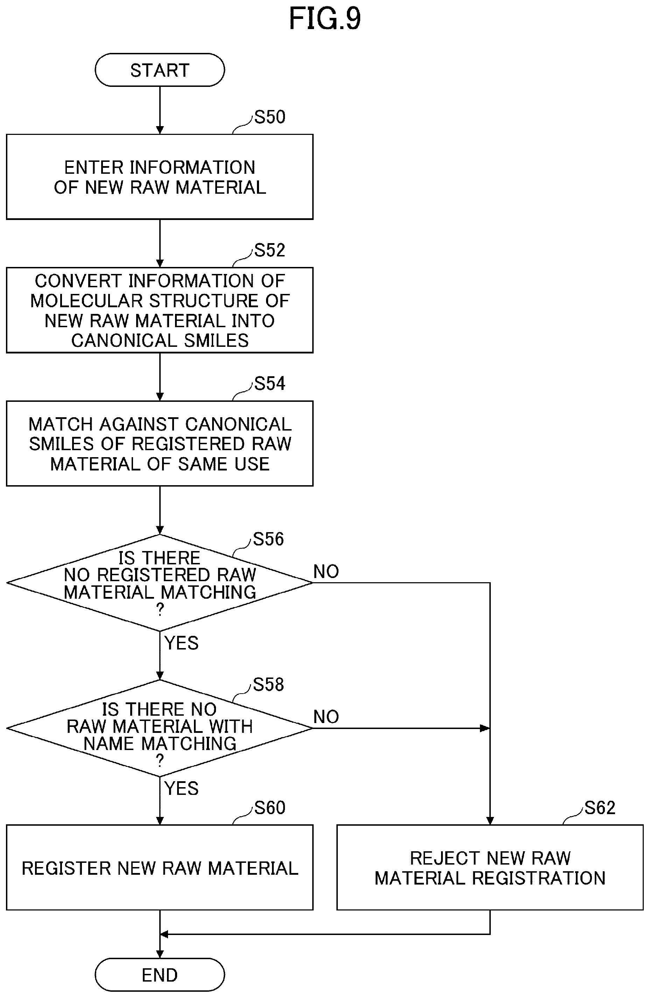

FIG. 9 is a flowchart illustrating an example of a process of prohibiting registration of a new raw material having an equivalent molecular structure. In step S50, the registration reception unit 24 of the design assistance device 10 receives an input of information of the new raw material from the new raw material registration screen 1000 illustrated in FIGS. 4 and 5.

In step S52, the registration reception unit 24 converts, into canonical SMILES of the new raw material, information of a molecular structure included in the information of the new raw material whose input has been received. In step S54, the registration reception unit 24 performs matching of the canonical SMILES of the new raw material against the canonical SMILES of the raw material registered in the raw material information storage 42 whose use is the same as that of the new raw material. The matching in terms of the canonical SMILES is just an example, and information used for matching may be any information as long as uniqueness of a raw material can be used for matching.

In step S56, the registration reception unit 24 determines whether or not there is a raw material registered in the raw material information storage 42 whose use is the same as that of the new raw material and whose canonical SMILES matches that of the new raw material.

If there is no raw material registered in the raw material information storage 42 whose use is the same as that of the new raw material and whose canonical SMILES matches that of the new raw material, the registration reception unit 24 proceeds to step S58. In step S58, the registration reception unit 24 performs matching of the name of the new raw material against the name of the raw material registered in the raw material information storage 42.

If there is no raw material registered in the raw material information storage 42 whose name matches the name of the new raw material, the registration reception unit 24 registers the new raw material in the raw material information storage 42 in step S60.

On the other hand, in step S56, if there is a raw material registered in the raw material information storage 42 whose use is the same as that of the new raw material and whose canonical SMILES matches that of the new raw material, the registration reception unit 24 performs the process of step S62.

In step S58, if there is a raw material registered in the raw material information storage 42 whose name matches the name of the new raw material, the registration reception unit 24 performs the process of step S62. In step S62, the registration reception unit 24 determines that the new raw material has a molecular structure equivalent to that of the raw material of the same use stored in the raw material information storage 42 or that the new raw material has a name the same as that of the raw material stored in the raw material information storage 42, and performs the process of step S62. In step S62, the registration reception unit 24 rejects registration of the new raw material in the raw material information storage 42 in order to prohibit duplicate registration of the new raw material having a molecular structure equivalent to or a name the same as that of the raw material of the same use stored in the raw material information storage 42.

### <<Assistance of Material Design through Direct Problem Analysis>>

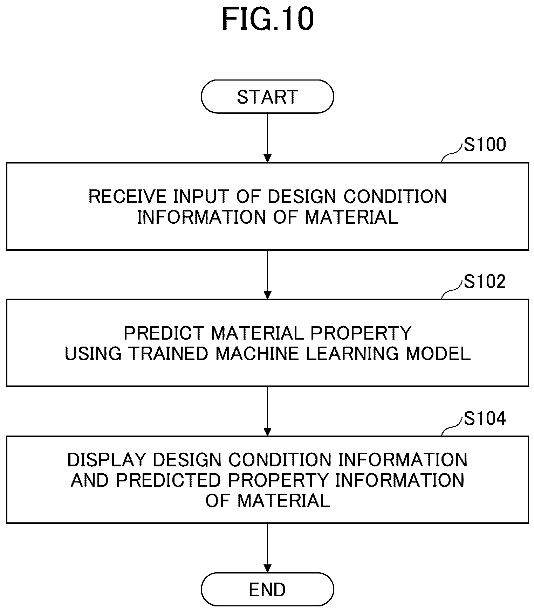

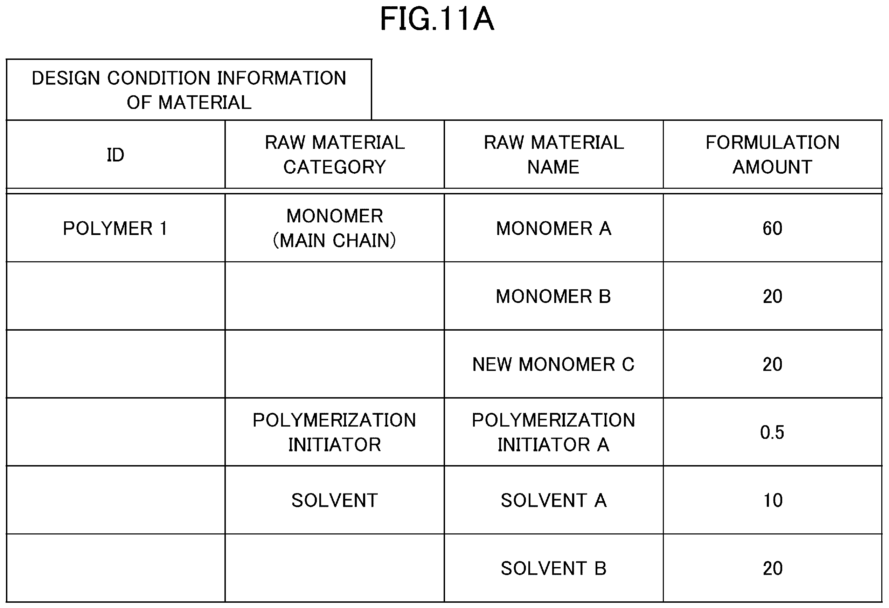

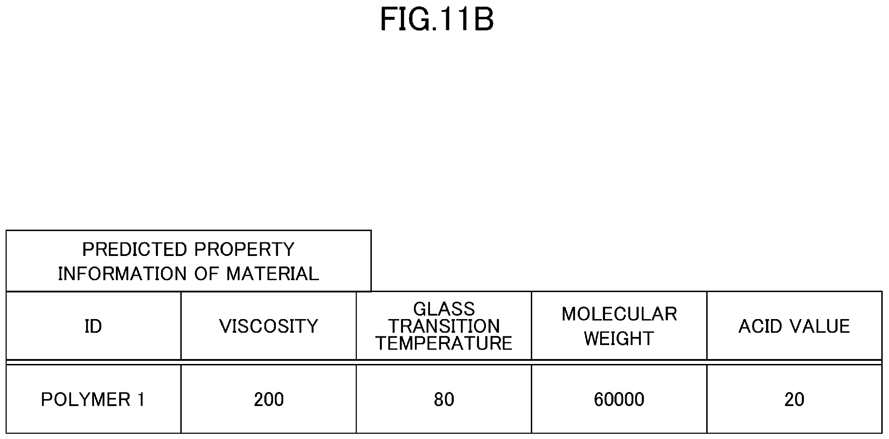

According to the information processing system 1 according to the present embodiment, for example, a process of assistance of material design through direct problem analysis as illustrated in FIG. 10 is performed by an operator selecting a menu of assistance of material design through direct problem analysis. FIG. 10 is a flowchart illustrating an example of a process of assistance of material design through direct problem analysis. FIG. 11A is a configuration diagram of an example of design condition information of a material. FIG. 11B is a configuration diagram of an example of predicted property information of a material.

In step S100, the design condition input reception unit 26 of the design assistance device 10 obtains, for example, design condition information of a material as illustrated in FIG. 11A, input by an operator with the user terminal 12. The design condition information of the material includes, as information, types and formulation amounts of raw materials stored in the raw material information storage 42. The ID "POLYMER 1" in FIG. 11A is an example of information identifying a material. The raw material category is an example of information indicating the use of a raw material. The raw material name is an example of information indicating the name of a raw material. The formulation amount is an example of information indicating the amount of a raw material used for synthesis of a material.

The raw material having the raw material name "NEW MONOMER C" in FIG. 11A is an example of the raw material registered in the raw material information storage 42 through new raw material registration. The raw materials other than the raw material name "NEW MONOMER C" in FIG. 11A are included as raw materials in the design condition information of the training dataset used for training of the machine learning model stored in the machine learning model storage 44.

In step S102, the controller 32 predicts, in the following manner, a property of the material, obtained by the design condition input reception unit 26 in step S100, by using the trained machine learning model stored in the machine learning model storage 44.

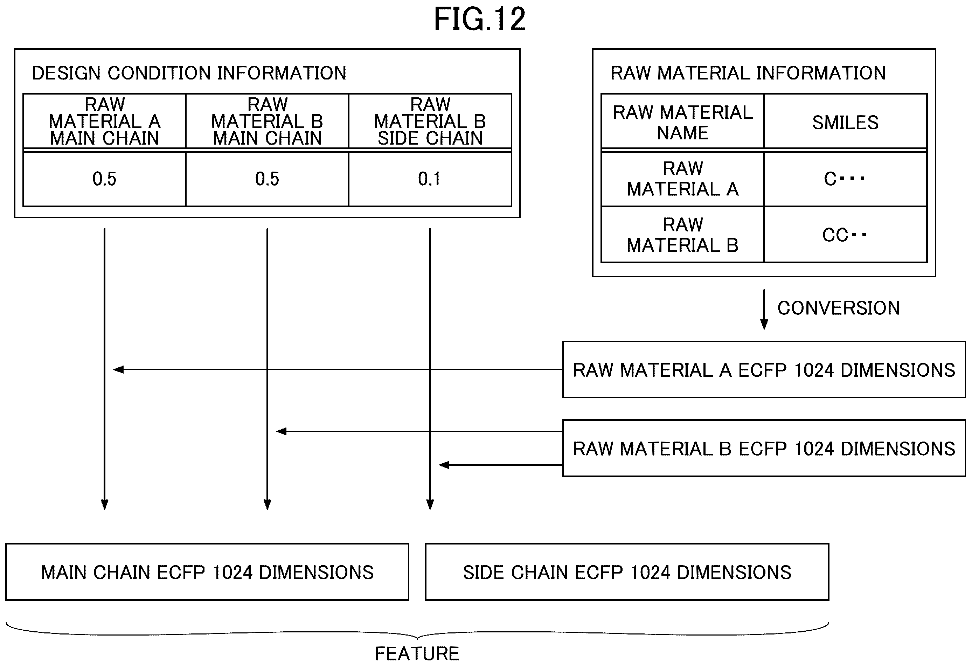

The controller 32 reads out the type and the formulation amount of a raw material to be formulated in a material, from the design condition information of the material obtained by the design condition input reception unit 26 in step S100, and provides the type and the formulation amount to the feature generation unit 34 and requests the feature generation unit 34 to generate a feature of the raw material. The feature generation unit 34 generates, for example, a feature of the raw material to be formulated in the material as illustrated in FIG. 12.

FIG. 12 is an explanatory diagram of an example of a process of generating a feature of a raw material to be formulated in a material. The feature generation unit 34 reads out, from the raw material information storage 42, information of a molecular structure of a raw material expressed in the SMILES notation, which is an example of the information of the molecular structure of "RAW MATERIAL A" and "RAW MATERIAL B" to be formulated in a material.

The feature generation unit 34 converts the information of the molecular structure of the raw material into numerical information by using a method of expressing the molecular structure of a raw material with numerical values, such as ECFP or the like. In FIG. 12, the information of the molecular structures of "RAW MATERIAL A" and "RAW MATERIAL B" to be formulated in the material is converted through ECFP into numerical information of 1024 dimensions.

The feature generation unit 34 generates a feature of the raw material to be input to the trained model by accumulating the numerical information of 1024 dimensions, obtained by converting the information of the molecular structures of "RAW MATERIAL A" and "RAW MATERIAL B" to be formulated in the material through ECFP, and the formulation amount of the raw material to be formulated in the material. For example, the information of the main chain as illustrated in FIG. 12 can generate a feature of raw materials of the main chain in accordance with: Σ (formulation amount of raw material i of the main chain × ECFP vector of raw material i of the main chain). In FIG. 12, "i = A, B".

As illustrated in FIG. 12, the information of the main chain and the information of the side chain are accumulated separately. This is because the same raw material can be used as an initiator or as a monomer, and thus accurate results cannot be obtained when accumulation is performed without considering applications of raw materials. When a material is formed of raw materials having no need to consider the order of reaction, calculation may be performed by accumulating all of the materials.

The controller 32 provides the material property prediction unit 38 with the feature of the raw material to be formulated in the material generated by the feature generation unit 34, and requests the material property prediction unit 38 to predict a property of the material. The material property prediction unit 38 inputs, into the trained machine learning model, the feature of the raw material to be formulated in the material, and predicts the property of the material. The material property prediction unit 38 obtains, for example, predicted property information of the material as illustrated in FIG. 11B, through property prediction of the material by using the trained machine learning model. The predicted property information of the material illustrated in FIG. 11B indicates a viscosity, a glass transition temperature, a molecular weight, and an acid value of the material, as examples of the properties of the material.

In step S104, the display controller 46 of the design assistance device 10 performs control to display, on the user terminal 12, the design condition information of the material, for example, as illustrated in FIG. 11A, input by the operator in step S100, and the predicted property information of the material, for example, as illustrated in FIG. 11B, obtained in step S102.

According to the process illustrated in FIG. 10, the property of the material containing a raw material not included in the training dataset used for training of the machine learning model can be predicted through direct problem analysis, and thus design of a material can be assisted.

### <<Assistance of Material Design through Inverse Problem Analysis>>

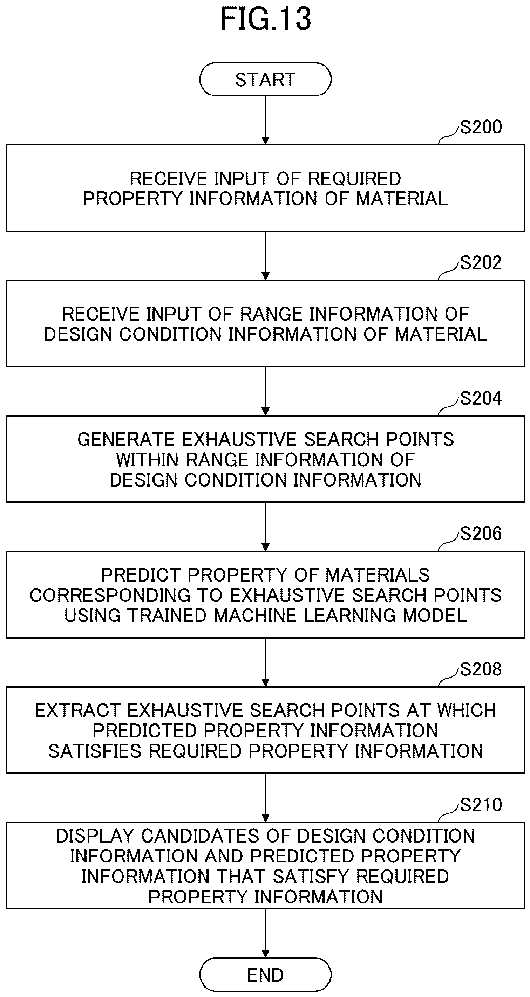

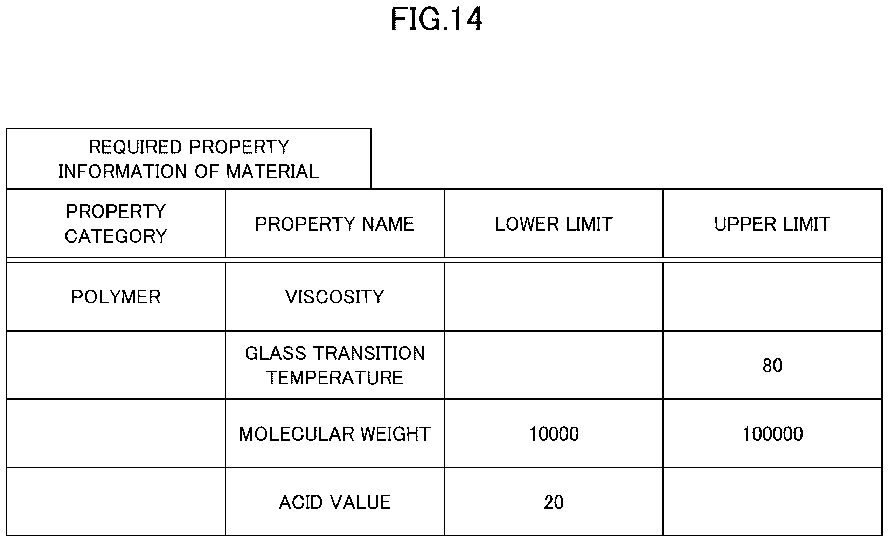

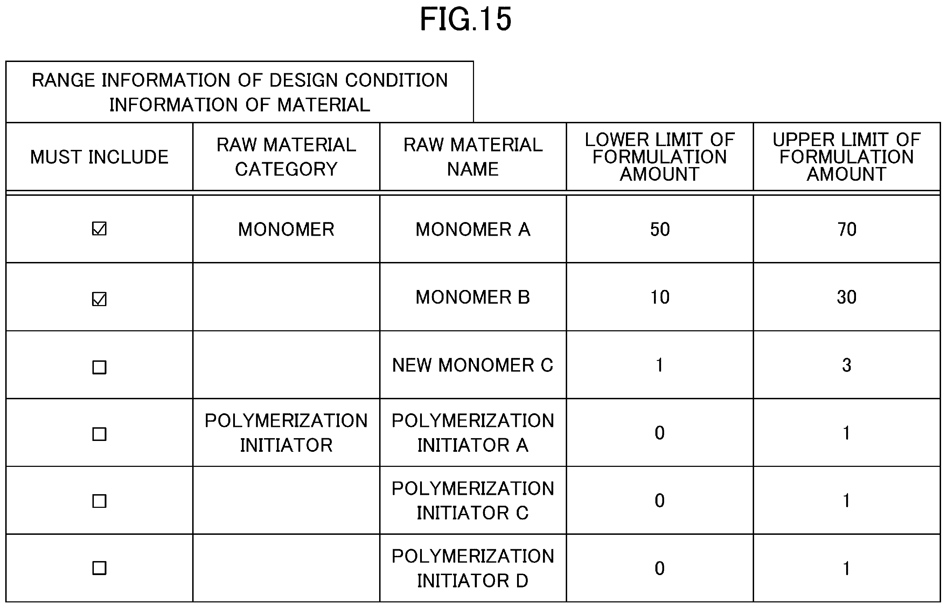

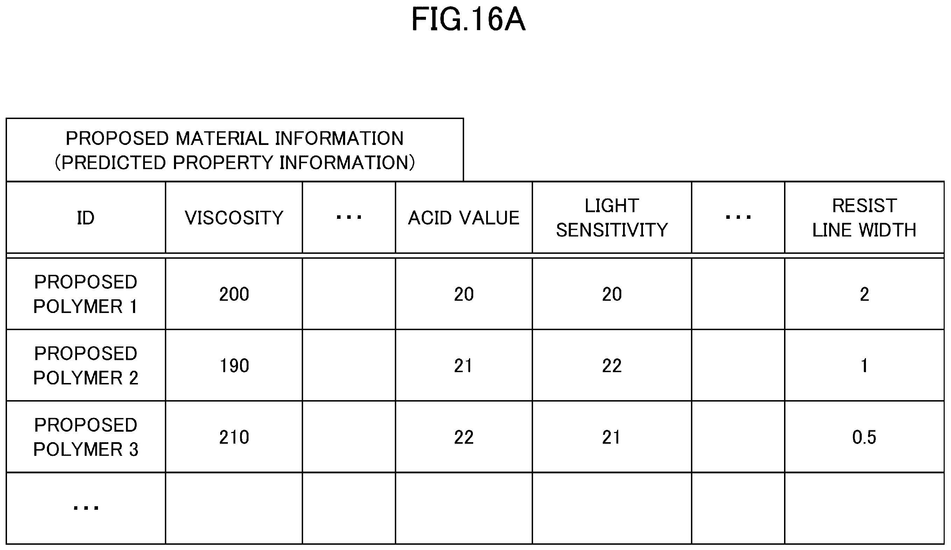

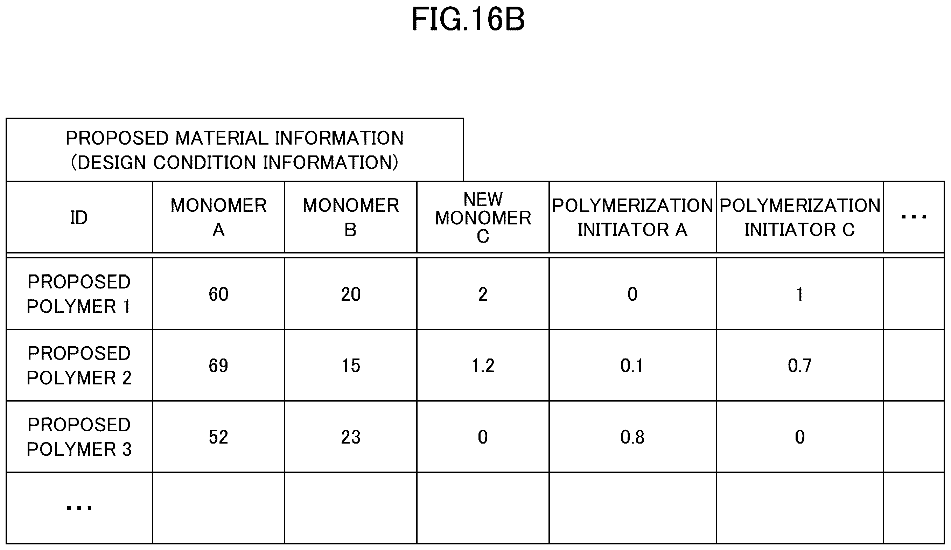

According to the information processing system 1 according to the present embodiment, for example, a process of assistance of material design through inverse problem analysis as illustrated in FIG. 13 is performed by an operator selecting a menu of assistance of material design through inverse problem analysis. FIG. 13 is a flowchart illustrating an example of a process of assistance of material design through inverse problem analysis. FIG. 14 is a configuration diagram of an example of required property information of a material. FIG. 15 is a configuration diagram of an example of range information of design condition information of a material. FIGS. 16A and 16B are configuration diagrams of an example of proposed material information.

In step S200, the required property input reception unit 28 of the design assistance device 10 obtains, for example, required property information of a material, as illustrated in FIG. 14, input by an operator with the user terminal 12. The required property information of a material illustrated in FIG. 14 is an example in which the required property of a material is expressed with lower and upper limits. As an example of the required property of a material, FIG. 14 illustrates lower and upper limits of a viscosity, a glass transition temperature, a molecular weight, and an acid value.

In step S202, the range information input reception unit 30 obtains, for example, range information of the design condition information of the material, as illustrated in FIG. 15, input by the operator with the user terminal 12. The range information of the design condition information of the material in FIG. 15 includes, as information, a raw material category, a raw material name, and the lower and upper limits of a formulation amount. The raw material category and the raw material name indicate the type of a raw material. The lower and upper limits of the formulation amount indicate a range of the formulation amount for each raw material.

The range information of the design condition information of the material in FIG. 15 is an example in which an operator can select a raw material that must be formulated in a material. The operator can select the raw material that must be formulated in the material by checking the item "MUST INCLUDE". FIG. 15 illustrates an example in which "MONOMER A" and "MONOMER B" are selected as a raw material that must be formulated. The raw material having the raw material name "NEW MONOMER C" in FIG. 15 is an example of the raw material that has been registered in the raw material information storage 42 through new raw material registration.

In step S204, the controller 32 provides the exhaustive search point generation unit 36 with the range information of the design condition information of the material obtained in step S202, and requests the exhaustive search point generation unit 36 to generate exhaustive search points within a range of the range information of the design condition information of the material. The exhaustive search point generation unit 36 generates a predetermined number (e.g., 1,000) of exhaustive search points within the range of the range information of the provided design condition information of the material.

In the case of the range information of the design condition information of the material as illustrated in FIG. 15, for example, the exhaustive search point is a combination of the formulation amounts of "MONOMER A", "MONOMER B", "NEW MONOMER C", "POLYMERIZATION INITIATOR A", "POLYMERIZATION INITIATOR C", and "POLYMERIZATION INITIATOR D", which are raw materials. The formulation amount of each raw material is selected from a range indicated by the item "LOWER LIMIT OF FORMULATION AMOUNT" and "UPPER LIMIT OF FORMULATION AMOUNT".

In step S206, the design condition proposal unit 40 predicts properties of the materials in accordance with a plurality of exhaustive search points generated in step S204 by using the trained machine learning model stored in the machine learning model storage 44, and obtains predicted property information of the materials corresponding to the plurality of exhaustive search points.

In step S208, the design condition proposal unit 40 extracts, for example, the exhaustive search points at which the predicted property information of the material obtained in step S206 satisfies the required property information of the material as illustrated in FIG. 14. The process of step S208 is a process of extracting the exhaustive search points at which the required property information of the material illustrated in FIG. 14 is satisfied among the exhaustive search points generated at random or at a predetermined step width within a range of design conditions.

In step S210, the display controller 46 of the design assistance device 10 performs control to display, on the user terminal 12, a candidate of the design condition information and the predicted property information of a material to be synthesized or the like in accordance with the exhaustive search points extracted in step S208, as information of a proposed material, for example, as illustrated in FIGS. 16A and 16B. For example, FIG. 16A illustrates, as information of the proposed material, the predicted property information of the material to be synthesized or the like in accordance with the exhaustive search points extracted in step S208. FIG. 16B illustrates, as information of the proposed material, the design condition information of the material to be synthesized or the like in accordance with the exhaustive search points extracted in step S208.

The information of the proposed material illustrated in FIGS. 16A and 16B is just an example. For example, the predicted property information of the proposed material illustrated in FIG. 16A and the design condition information of the proposed material illustrated in FIG. 16B may be combined together using the ID as a key, and aligned in a single row for display.

According to the process of FIG. 13, among materials containing a raw material not included in the training dataset used for training of the machine learning model, the design condition information of the material that satisfies required properties can be proposed through inverse problem analysis, and thus material design can be assisted.

### [Other Embodiments]

A material designed by the design assistance device 10 according to the present embodiment may be produced, for example, by a production device configured to produce a material by formulating a plurality of raw materials. Specifically, design condition information of a material may be supplied to the production device, and the production device is caused to produce a material by formulating a plurality of raw materials.

According to the information processing system 1 according to the present embodiment, it is possible to provide a design assistance device, a design assistance method, a program, and an information processing system that assist design of a material by predicting a property of the material in which a plurality of raw materials are to be formulated.

Although the present embodiment has been described above, it should be understood that various changes in terms of forms and details are possible without departing from the intent and scope of the claims recited. The present invention has been described above with reference to the examples, but the present invention is not limited to the above examples, and various modifications are possible within the scope of the claims recited. The present application claims priority to the basic application No. 2022-149308 filed with the Japan Patent Office on September 20, 2022, the entire contents of which are incorporated herein by reference.

### REFERENCE SIGNS LIST

- 1: Information processing system
- 10: Design assistance device
- 12: User terminal
- 18: Communication network
- 24: Registration reception unit
- 26: Design condition input reception unit
- 28: Required property input reception unit
- 30: Range information input reception unit
- 32: Controller
- 34: Feature generation unit
- 36: Exhaustive search point generation unit
- 38: Material property prediction unit
- 40: Design condition proposal unit
- 42: Raw material information storage
- 44: Machine learning model storage
- 46: Display controller

## Claims

1. A design assistance device, which is configured to assist design of a material in which a plurality of raw materials are to be formulated, by using a machine learning model that has learned a correspondence relationship between design condition information of a material, in which a plurality of raw materials are to be formulated, and property information of the material, the design assistance device comprising:
a raw material information storage configured to store information of names and attributes of a plurality of registered raw materials;
a registration reception unit configured to receive an input of information of a name and an attribute of a raw material to be newly registered, and store the received information in the raw material information storage; and
a feature generation unit configured to generate a feature of the raw material that is inputtable to the machine learning model, the feature being generated from information that is selected as the design condition information and is information of attributes of a plurality of raw materials stored in the raw material information storage.

2. The design assistance device according to according to claim 1, wherein
the registration reception unit is configured to prohibit new registration of a raw material that matches information of a molecular structure of the raw material stored in the raw material information storage.

3. The design assistance device according to claim 1, wherein
the information of the attribute of the raw material includes information of use of the raw material, and
the registration reception unit is configured to prohibit new registration of a raw material that matches information of a molecular structure of the raw material and information of the use of the raw material, which are stored in the raw material information storage.

4. The design assistance device according to claim 3, wherein
the registration reception unit is configured to prohibit new registration of a raw material that matches the name of the raw material stored in the raw material information storage.

5. The design assistance device according to any one of claims 1 to 4, wherein
the registration reception unit is configured to receive an input of information of a molecular structure of a raw material in accordance with a SMILES notation, a SMARTS notation, or an InChI notation, as the information of the attribute of the raw material to be newly registered.

6. The design assistance device according to any one of claims 1 to 4, wherein
the registration reception unit is configured to receive an input of information of a molecular structure of a raw material in an MOL format, an SDF format, a PDB format, or a CIF format, as the information of the attribute of the raw material to be newly registered.

7. The design assistance device according to any one of claims 1 to 6, wherein
the feature generation unit is configured to generate the feature of the raw material from information of a molecular structure of the raw material by using a fingerprint method.

8. The design assistance device according to any one of claims 1 to 7, wherein
the feature generation unit is configured to generate the feature of the raw material by calculating a physical property value of a molecule from a molecular structure of the raw material.

9. The design assistance device according to any one of claims 1 to 8, wherein
the information of the attribute whose input is received by the registration reception unit includes a physical property value of the raw material.

10. The design assistance device according to any one of claims 1 to 9, wherein
the design assistance device includes
a design condition input reception unit configured to receive an input of the design condition information that includes information of a plurality of raw materials stored in the raw material information storage, and
a material property prediction unit configured to predict a property of the material using the machine learning model in accordance with the design condition information whose input has been received.

11. The design assistance device according to any one of claims 1 to 9, further comprising:
a required property input reception unit configured to receive an input of required property information of the material;
a range information input reception unit configured to receive an input of range information of the design condition information that includes information of a plurality of raw materials stored in the raw material information storage; and
a design condition proposal unit configured to propose a candidate of the design condition information that satisfies the required property information, from a result obtained by predicting a property of the material using the machine learning model in accordance with the design condition information within a range of the range information.

12. The design assistance device according to claim 10 or 11, further comprising:
a display controller configured to display a predicted property of the material, or a proposed candidate of the design condition information.

13. A design assistance method, in which a computer assists design of a material in which a plurality of raw materials are to be formulated, by using a machine learning model that has learned a correspondence relationship between design condition information of a material, in which a plurality of raw materials are to be formulated, and property information of the material, the design assistance method comprising:
a registration reception step of receiving an input of information of a name and an attribute of a raw material to be newly registered, and registering the received information in a raw material information storage that stores information of names and attributes of a plurality of registered raw materials; and
a feature generation step of generating a feature of the raw material that is inputtable to the machine learning model, the feature being generated from information that is selected as the design condition information and is information of attributes of a plurality of raw materials stored in the raw material information storage.

14. A program causing a computer to execute, the computer assisting design of a material in which a plurality of raw materials are to be formulated, by using a machine learning model that has learned a correspondence relationship between design condition information of a material, in which a plurality of raw materials are to be formulated, and property information of the material:
a registration reception procedure of receiving an input of information of a name and an attribute of a raw material to be newly registered, and registering the received information in a raw material information storage that stores information of names and attributes of a plurality of registered raw materials; and
a feature generation procedure of generating a feature of the raw material that is inputtable to the machine learning model, the feature being generated from information that is selected as the design condition information and is information of attributes of a plurality of raw materials stored in the raw material information storage.

15. An information processing system, in which a plurality of computers assist design of a material in which a plurality of raw materials are to be formulated, by using a machine learning model that has learned a correspondence relationship between design condition information of a material, in which a plurality of raw materials are to be formulated, and property information of the material, the design assistance system comprising:
a raw material information storage configured to store information of names and attributes of a plurality of registered raw materials;
a registration reception unit configured to receive an input of information of a name and an attribute of a raw material to be newly registered, and store the received information in the raw material information storage; and
a feature generation unit configured to generate a feature of the raw material that is inputtable to the machine learning model, the feature being generated from information that is selected as the design condition information and is information of attributes of a plurality of raw materials stored in the raw material information storage.
